Europäisches Patentamt

European Patent Office

Office européen des brevets

(19)

(11) Veröffentlichungsnummer: **0 236 791**

**A1**

(12) **EUROPÄISCHE PATENTANMELDUNG**

(21) Anmeldenummer: 87102157.2

(22) Anmeldetag: 16.02.87

(51) Int. Cl.⁴: **G21C 17/02** , G01N 1/22 , G01N 33/18

(30) Priorität: 27.02.86 DE 3606457

(43) Veröffentlichungstag der Anmeldung:
16.09.87 Patentblatt 87/38

(84) Benannte Vertragsstaaten:
DE FR

(71) Anmelder: **KRAFTWERK UNION AKTIENGESELLSCHAFT**
**Wiesenstrasse 35**
**D-4330 Mülheim (Ruhr)(DE)**

(72) Erfinder: **Emmert, Hermann**
**Hildensee Strasse 5**
**D-8801 Burgbernheim(DE)**

(74) Vertreter: **Mehl, Ernst, Dipl.-Ing. et al**
**Postfach 22 01 76**
**D-8000 München 22(DE)**

(54) **Einrichtung zum Messen von in Wasser gelösten Gasen.**

(57) Die Erfindung betrifft eine Einrichtung zum Messen von in Wasser gelösten Gasen mit einem Gasaustauscher, dem ein Trägergas zuführbar ist. Das ausgewaschene Gas wird Analyse-(7) und Meßgeräten (8) zugeführt. Es ist vorgesehen, daß der Gasaustauscher eine in senkrechter Lage angeordnete Füllkörperkolonne (1) ist, deren Wasserableitung (4) mit einem Sammelbehälter (10) in Verbindung steht. Von der Wasserableitung (4) zweigt eine Probenleitung (41) ab. Zum Druckausgleich ist in der Wasserableitung (4) entweder ein Druckregler (43) oder ein Siphon (44) angeordnet. Ein zweiter Zweig der Gasableitung (5) geht von den Analyse-- (7) und Meßgeräten (8) aus und ist gleichfalls mit dem Sammelbehälter (10) verbunden, der in eine Schutzgasleitung (13) eingefügt ist. Falls in einem Kernkraftwerk Primärwasser entgast werden soll, sind die Füllkörperkolonne (1) und der Sammelbehälter (10) zum Abschirmen hinter einer vorhandenen Strahlenschutzwand (15) angeordnet, durch die nur dünne Zu-und Ableitungen hindurchgeführt sind.

FIG 1

## Einrichtung zum Messen von in Wasser gelösten Gasen

Die Erfindung betrifft eine Einrichtung zum Messen von in Wasser gelösten Gasen mit einem Gasaustauscher, dem über eine Wasserzuleitung das zu untersuchende Wasser und über eine Gaszuleitung ein Trägergas zuführbar ist und wobei von dem Gasaustauscher außer einer Wasserableitung für entgastes Wasser eine Gasableitung für ausgewaschenes Gas ausgeht, die über einen Gastrockner mit Analyse und Meßgeräten verbunden ist.

Eine derartige Einrichtung ist aus der DE-OS 33 36 423 bekannt. Dabei wird dem Kontaktzelle genannten Gasaustauscher als Trägergas Argon zugeführt, das dann im Probenwasser vorhandene Gase mitnimmt. Dieses Gasgemisch wird getrocknet und anschließend in einer Trennsäule chromatographisch getrennt. Über Wärmeleitfähigkeitsdetektoren werden die ausgewaschenen Gase quantitativ erfaßt.

Die bekannte Kontaktzelle besteht aus einem zylindrischen Teil, in dem spiralförmig gewundene Rohre verlaufen. Die beiden Rohre, von denen das eine das zu untersuchende Wasser und das andere Argon führt, weisen gleichmäßig über den Umfang verteilte Öffnungen auf. Dadurch kommen Wasser und Argon in Kontakt und die im Wasser gelösten Gase werden durch das Argon ausgewaschen. Mit einem Flansch am Gehäuse ist eine Gasab fuhrleitung verbunden, durch die das ausgewaschene Gas beispielsweise einem Gaschromatographen zuführbar ist.

In einer derartigen Kontaktzelle erfolgt der Gasaustausch nur bis zu einem zunächst unbekannten Gleichgewicht. Die Lage dieses Gleichgewichtes und damit der Wirkungsgrad der Einrichtung muß durch Kalibrierung ermittelt werden.

Da eine Einrichtung zum Auffangen des Abwassers und des Abgases fehlt, ist die bekannte Einrichtung zur Überwachung von kontaminiertem Wasser ungeeignet. Beispielsweise sind aber im Primärkühlmittel von Leicht-oder Schwerwasserreaktoren verschiedenartige Gase gelöst, für die eine Konzentrationsüberwachung wünschenswert ist. Durch Radiolyse von Wasser kann Wasserstoff und Sauerstoff entstehen. In Druckwasserreaktoren wird die Radiolyse des Primärkühlmittels begrenzt. Dabei müssen jedoch die Grenzwerte der Wasserstoff-und Sauerstoffkonzentration überwacht werden. Insbesondere die Konzentration des Sauerstoffes, der für Korrosion verantwortlich ist, muß überwacht werden. Ebenso sind die Stickstoffanteile im Primärkühlmittel zu überwachen. Stickstoff ist für die Brennelementkorrosion mit verantwortlich. Bei Brennelementschäden treten die Edelgase Krypton und Xenon in das Primärkühlmittel über. Deren Konzentrationswerte lassen Aussagen über den Zustand der Brennelemente zu.

Der Erfindung liegt die Aufgabe zugrunde, eine Einrichtung zum Messen von in Wasser gelösten Gasen zu entwickeln, die ohne Kalibrierung in der Flüssigkeitsphase auskommt. Zur Untersuchung kontaminierter Flüssigkeiten, insbesondere des Primärkühlmittels von Leicht-und Schwerwasserreaktoren sollen alle kontaminierten Flüssigkeiten und Gase in ein geschlossenes System einzuspeisen sein.

Unabhängig von dem in dem geschlossenen System herrschenden Druck soll es möglich sein, eine Probe des entgasten Wassers zu entnehmen.

Die Aufgabe wird erfindungsgemäß dadurch gelöst, daß der Gasaustauscher eine in senkrechter Lage angeordnete Füllkörperkolonne ist, an der die Wasserzuleitung und die Gasableitung oben und die Gaszuleitung und die Wasserableitung unten angeschlossen sind, daß die Wasserableitung mit einem Sammelbehälter verbunden ist, daß mit den Analyse-und Meßgeräten ein zweiter Zweig der Gasableitung für das nicht mehr benötigte Gas verbunden ist, der mit dem Sammelbehälter in Verbindung steht und daß von der Wasserableitung abzweigend eine Probenleitung mit Ablaßhahn zur Entnahme von Wasserproben, die von Gasen befreit sind, angeordnet ist.

Eine derartige Füllkörperkolonne hat einen nahezu 100%igen Wirkungsgrad. Durch ihren erfindungsgemäßen Einsatz wird der Vorteil erzielt, daß eine aufwendige und häufig zu wiederholende Kalibrierung des Gasaustauschers mit einer Eichlösung überflüssig ist. Es reicht aus, die der Füllkörperkolonne nachgeschalteten Analyse-und Meßgeräte in regelmäßigen Zeitabständen mit Prüfgas zu kalibrieren.

Prüfgas mit einer bekannten Zusammensetzung ist im Handel erhältlich und wird beispielsweise über ein Dreiwegeventil vor den Analyse-und Meßgeräten in die Gasableitung eingespeist.

Neben der einfachen Kalibrierbarkeit wird mit der erfindungsgemäßen Einrichtung der Vorteil erzielt, daß Ab wasser wie auch Abgas einem Sammelbehälter zugeführt werden und dadurch bei einer Untersuchung von radioaktiven Wässern ein unkontroliertes Entweichen radioak-tiver Stoffe verhindert wird. In einem Kernkraftwerk ist der Sammelbehälter beispielsweise in das interne Abgassystem eingebunden. Er nimmt also nur Abwasser auf. Das Abgas wird im Kraftwerk weiterbehandelt.

Von der Wasserableitung der Füllkörperkolonne abzweigend ist eine Probenleitung mit Ablaßhahn angeordnet. Damit wird die Entnahme von gereinigten, von radioaktiven Gasen befreiten Wasserproben ermöglicht. Da jedoch der Druck im Sammelbehälter oft niedriger ist als der Außendruck, ist eine Probenentnahme nur mit zusätzlichen Mitteln durchführbar. Beispielsweise ist in der Wasserableitung hinter dem Abzweig der Probenleitung ein Druckregler angeordnet und von der Gasableitung zweigt eine Bypaßgasleitung ab, die mit der Wasserableitung unmittelbar vor dem Druckregler verbunden ist. Die Gasleitung für das untersuchte Gas ist mit der Wasserableitung hinter dem Druckregler verbunden.

Durch den Einsatz des Druckreglers in Verbindung mit der Bypaßgasleitung wird der Vorteil erzielt, daß in der Probenleitung stets ein Druck herrscht, der größer ist als der Außendruck. Dadurch ist das Abfließen der Wasserproben bei geöffnetem Ablaßhahn gewährleistet.

Ein problemloses Abfließen der Wasserproben aus der Probenleitung ist nach einem anderen Beispiel dadurch gewährleistet, daß die Wasserableitung schleifenförmig nach der Art eines Siphons ausgebildet ist. Die Probenleitung zweigt von der Wasserableitung niveaugleich mit dem unteren Bogen ab. Der mittlere Siphonschenkel der Wasserableitung ist höher als eine Wassersäule, die die Differenz zwischen Außendruck und dem Druck im Sammelbehälter erzeugt. Auch durch diese Anordnung wird gewährleistet, daß der Druck in der Wasserableitung stets höher ist als der Außendruck, wodurch eine Probenentnahme jederzeit möglich ist.

Die Füllkörperkolonne, der Gastrockner, der Sammelbehälter und die diese verbindenden Leitungen sind beispielsweise zum Abschirmen hinter einer vorhandenen Strahlenschutzwand angeordnet. Dann werden nur relativ dünne Zu-und Ableitungen für Gas und Wasser durch die Strahlenschutzwand hindurchgeführt. Hiermit wird der Vorteil erzielt, daß bei einer Untersuchung kontaminierter Wasserproben, die beispielsweise aus dem Primärkreislauf eines Kernkraftwerkes stammen, die Strahlenbelastung im Bereich der Analyse-und Meßgeräte gering ist. Diese Geräte sind dann ohne Gefahr zu bedienen und abzulesen.

Als Gastrockner ist beispielsweise ein Gaskühler eingesetzt. Das dort anfallende Kondensat wird über eine Leitung, z.B. über die Bypaßgasleitung abgeführt, die mit der Wasserableitung der Füllkörperkolonne verbunden ist. Dadurch wird auch das möglicherweise kontaminierte Kondensat in den Sammelbehälter eingespeist.

Die Füllkörperkolonne besteht aus einem Rohr, das in einem festgelegten Bereich mit Füllkörpern, beispielsweise mit Drahtspiralen, zur Oberflächenvergrößerung angefüllt ist. Das der Füllkörperkolonne zugeführte Wasser bildet einen Flüssigkeitsfilm auf der gesamten großen Oberfläche. Dadurch wird der Austauschprozeß zwischen Gas und Flüssigkeit begünstigt.

Im mittleren Bereich des mit Füllkörpern angefüllten Rohrabschnittes befindet sich nach der Erfindung ein Trichter, dessen Öffnungsrand mit der Rohrinnenwand dicht abschließt. Dadurch wird der Vorteil erzielt, daß zunehmend im Randbereich des Rohres fließendes Wasser in das Zentrum des Füllkörperabschnittes zurückgeleitet wird, wodurch eine gleichmäßige Benetzung aller Füllkörper gewährleistet ist. Der Trichter trägt zum hohen Wirkungsgrad der Füllkörperkolonne bei.

Als Analyse-und Meßgeräte sind verschiedenartige Geräte einsetzbar. Ein Analysegerät zur Untersuchung des ausgewaschenen Gases ist beispielsweise ein Gaschromatograph, der mit einem Wärmeleitfähigkeitsdetektor verbunden ist. Hiermit werden die Bestandteile des Gases bestimmt.

Zum Einbau in die Gasleitung für das zu untersuchende Gas sind als Meßgeräte ein Strahlungsdetektor zum Messen der Radioaktivität der Gase und ein Vielkanalanalysator zur nuklidspezifischen Messung besonders geeignet.

Beispielsweise sind hintereinander ein Gaschromatograph mit einem Wärmeleitfähigkeitsdetektor und ein Strahlendetektor angeordnet. Mit den genannten Analyse-und Meßgeräten, die an sich bekannt sind, erhält man notwendige Informationen über die im Wasser gelösten Gase.

Mit der Erfindung wird der Vorteil erzielt, daß die Einrichtung zum Messen von in Wasser gelösten Gasen stets zuverlässig arbeitet und einfach zu kalibrieren ist. Darüber hinaus ist die erfindungsgemäße Einrichtung besonders für die Überwachung kontaminierten Wassers, wie des Primärkühlmittels von Kernkraftwerken geeignet, da nur eine minimale Strahlenbelastung für das Personal vorhanden ist. Alle kontaminierten Flüssigkeiten und Gase sind in ein geschlossenes System rückführbar. Außerdem gewährleistet die erfindungsgemäße Einrichtung, daß jederzeit von radioaktiven Gasen befreites Wasser für weitere Untersuchungen aus dem System ent nommen werden kann.

Die Meß-und Analysegeräte befinden sich außerhalb des möglicherweise kontaminierten Bereiches. Es sind wahlweise manuell oder automatisch zu betreibende Geräte einsetzbar.

Die Erfindung wird anhand der Zeichnung näher erläutert:

Fig. 1 zeigt die erfindungsgemäße Einrichtung zum Messen von in Wasser gelösten Gasen mit einem Druckregler in der Wasserableitung.

Fig. 2 zeigt einen Teil der erfindungsgemäßen Einrichtung mit einem Siphon in der Wasserableitung.

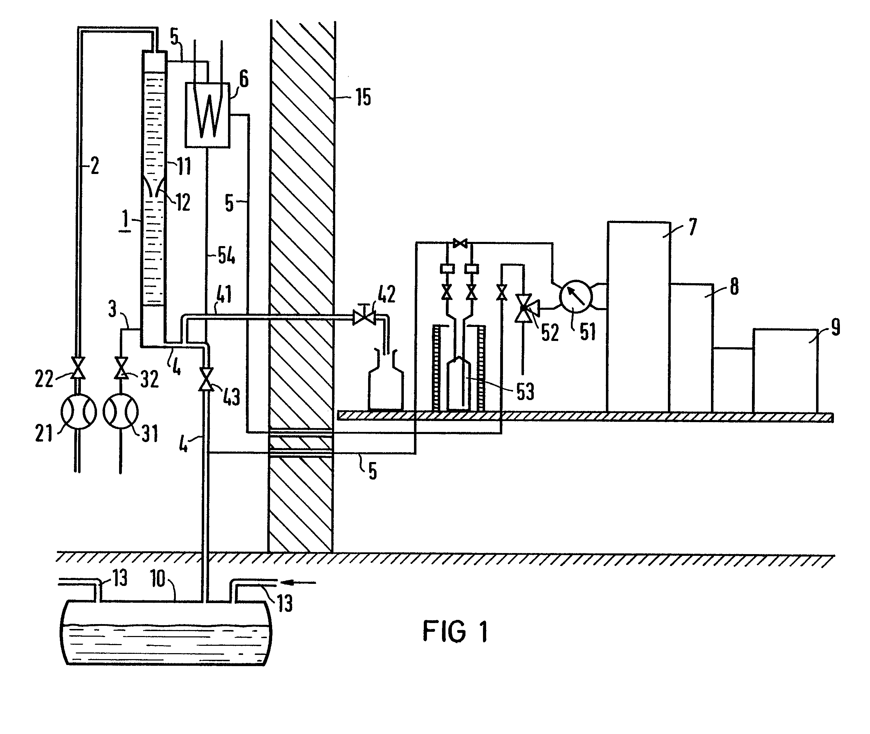

Die Einrichtung zum Messen von in Wasser gelösten Gasen nach Fig. 1 weist als Gasaustauscher eine rohrförmige Füllkörperkolonne 1 auf. Eine Wasserzuleitung 2, in der ein Durchflußmesser 21 und ein Durchflußregler 22 angeordnet sind, ist mit der senkrecht angeordneten Füllkörperkolonne 1 an einem oberen Anschlußflansch verbunden. Durch die Wasserzuleitung 2 wird zu untersuchendes Wasser, das beispielsweise aus dem Primärkreislauf eines Kernkraftwerkes kommt, mit konstantem Volumenstrom zugeführt. Eine Gaszuleitung 3, in der ein Durchflußmesser 31 und ein Durchflußregler 32 angeordnet sind, steht mit der Füllkörperkolonne 1 an einem unteren Anschlußflansch in Verbindung. Durch diese Gaszuleitung 3 wird mit konstantem Volumenstrom ein Trägergas, beispielsweise Argon zugeführt, durch das in dem zu untersuchenden Wasser gelöste Gase ausgewaschen werden. Am unteren Teil der Füllkörperkolonne 1 ist eine Wasserableitung 4 für das entgaste Wasser angeschlossen. Vom oberen Teil der Füllkörperkolonne 1 geht eine Gasableitung 5 für das ausgewaschene Gas aus.

Ein bestimmter Rohrabschnitt 11 der Füllkörperkolonne 1 ist mit dichtgepackten Füllkörpern, beispielsweise mit Drahtspiralen angefüllt. Die dadurch erzielte Oberflächenvergrößerung in der Füllkörperkolonne 1 verbessert deren Wirkungsgrad. Damit alle Füllkörper der Packung gleichmäßig vom zu untersuchenden Wasser benetzt werden, ist im mittleren Bereich des mit Füllkörpern gefüllten Rohrabschnittes 11 ein Trichter 12 angeordnet, dessen Öffnungsrand mit der Rohrinnenwand der Füllkörperkolonne 1 dicht abschließt. Dadurch wird das zunehmend im äußeren Bereich des Rohrquerschnittes fließende Wasser wieder zur Mitte geleitet.

Die Gasableitung 5 der Füllkörperkolonne 1 ist über einen Gaskühler 6, in dem das ausgewaschene Gas getrocknet wird, mit einem Gaschromatographen 7 verbunden. Vor dem Gaschromatograph 7 ist in die Gasableitung 5 ein Dosierventil 51 und ein Dreiwegeventil 52 zum Einspeisen von Prüfgas eingefügt, das zur Kalibrierung der Meß-und Analysegeräte dient.

Mit dem Gaschromatograph 7 ist beispielsweise ein Beta-strahlen-Detektor 8 verbunden, der die Betaaktivität der Gasbestandteile bestimmt. Zur Auswertung der Meßergebnisse von Gaschromatograph 7 und Betastrahlen-Detektor 8 ist mit deren Signalausgängen eine Auswerteeinheit mit Schreiber 9 verbunden.

Vom Gaschromatograph 7 und vom Dosierventil 51 ausgehend ist die Gasableitung 5, an der ein Gasprobenbehälter 53 anschließbar ist, weitergeführt.

Die Wasserableitung 4 der Füllkörperkolonne 1 ist mit einem Sammelbehälter 10 verbunden, durch den über eine kraftwerkinterne Leitung 13 ein Schutzgas geführt wird. Von der Wasserableitung 4 zweigt eine mit einem Ablaß hahn 42 versehene Probenleitung 41 ab zur Entnahme von Wasserproben in einen Probenbehälter.

Da im Sammelbehälter 10 ein geringer Unterdruck herrschen kann, sind zusätzliche Einrichtungen notwendig, damit Wasserproben gegen Außendruck entnehmbar sind. Nach Fig. 1 ist in der Wasserableitung 4 dazu zwischen dem Abzweig der Probenleitung 41 und dem Sammelbehälter 10 ein Druckregler 43 angeordnet. Von der Gasableitung 5 zweigt am Gastrockner 6 eine Bypaßleitung 54 ab, die vor dem Druckregler 43 in die Wasserableitung 4 einmündet. Dadurch ist ein Druck, der größer als der Außendruck ist, in der Probenleitung 41 gewährleistet und eine Probennahme ist stets möglich.

Das bereits untersuchte und analysierte Gas, das nicht mehr gebraucht wird, wird über den zweiten Zweig der Gasableitung 5, der mit der Wasserableitung 4 hinter dem Druckregler 43 verbunden ist, zusammen mit dem entgasten Wasser in den Sammelbehälter 10 eingespeist. Dort wird das Gas mit dem Schutzgas weggespült.

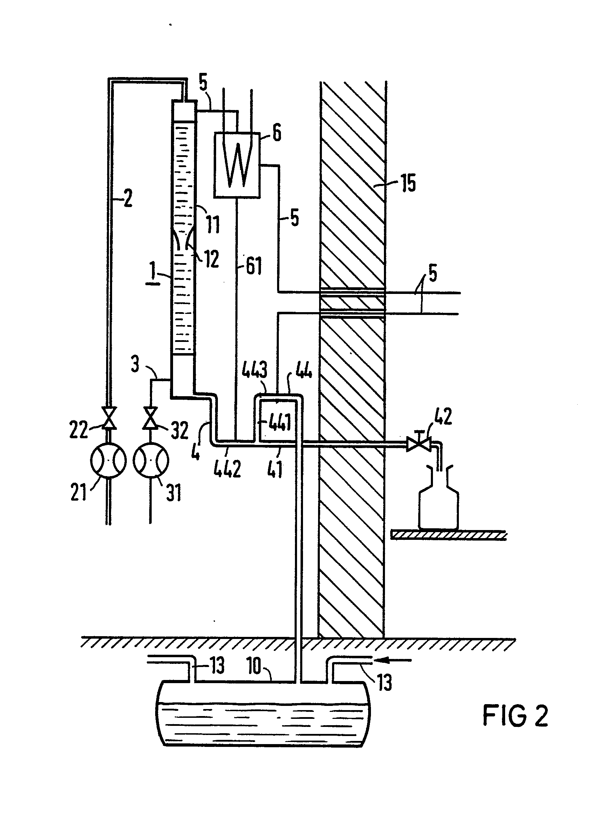

Um einen ausreichend hohen Druck in der Probenleitung 41 sicherzustellen, ist nach Fig. 2 die Wasserableitung 4 schleifenförmig nach der Art eines Siphons 44 angeordnet. Die Probenleitung 41 zweigt von der Wasserableitung 4 niveaugleich mit dem unteren Bogen 442 des Siphons 44 ab. Der obere Bogen 443 des Siphons 44 ist so hoch oberhalb des unteren Bogens 442 angeordnet, daß der mittlere Siphonschenkel 441 höher ist als eine Wassersäule, deren Druck der Differenz zwischen Außendruck und dem Druck im Sammelbehälter 10 entspricht.

Der zweite Zweig der Gasableitung 5 mündet nach Fig. 2 in den oberen Bogen 443 des Siphons 44 ein. Im Gas kühler 6 anfallendes Kondensat wird über eine Kondensatleitung 61 in die Wasserableitung 4 eingespeist. In der Ausführungsform nach Fig. 1 übernimmt die Bypaßgasleitung 54 die Funktion einer Kondensatleitung.

Falls radioaktiv kontaminiertes Wasser zu untersuchen ist, ist die erfindungsgemäße Einrichtung nach beiden Fig. so angeordnet, daß die Füllkörperkolonne 1, der Gaskühler 6, der Sammelbehälter 10 und die diese verbindenden Leitungen zum Abschirmen hinter einer vorhandenen Strahlenschutzwand 15 angeordnet sind. Durch diese Strahlenschutzwand 15 sind nur dünne Zu- und Ableitungen für Gas und Wasser hindurchgeführt. Die geschilderte Einrichtung eignet sich insbesondere, weil in Kernkraftwerken der Sammelbehälter 10 und die Strahlenschutzwand 15 bereits vorhanden sind, für die Untersuchung von Primärkühlwasser. Falls das zu untersuchende Wasser jedoch inaktiv ist, kann auf eine Einleitung in einen Sammelbehälter 10 und auf eine Strahlenschutzwand 15 verzichtet werden.

**Ansprüche**

1. Einrichtung zum Messen von in Wasser gelösten Gasen mit einem Gasaustauscher, dem über eine Wasserzuleitung (2) das zu untersuchende Wasser und über eine Gaszuleitung (3) ein Trägergas zuführbar ist und wobei von dem Gasaustauscher außer einer Wasserableitung (4) für entgastes Wasser eine Gasableitung (5) für ausgewaschenes Gas ausgeht, die über einen Gastrockner mit Analyse und Meßgeräten verbunden ist,
**dadurch gekennzeichnet,**
daß der Gasaustauscher eine in senkrechter Lage angeordnete Füllkörperkolonne (1) ist, an der die Wasserzuleitung (2) und die Gasableitung (5) oben und die Gaszuleitung (3) und die Wasserableitung (4) unten angeschlossen sind, daß die Wasserableitung (4) mit einem Sammelbehälter (10) verbunden ist, daß mit den Analyse- und Meßgeräten ein zweiter Zweig der Gasableitung (5) für das nicht mehr benötigte Gas verbunden ist, der mit dem Sammelbehälter (10) in Verbindung steht und daß von der Wasserableitung (4) abzweigend eine Probenleitung (41) mit Ablaßhahn (42) zur Entnahme von Wasserproben, die von Gasen befreit sind, angeordnet ist.
2. Einrichtung nach Anspruch 1,
**dadurch gekennzeichnet,**
daß in der Wasserableitung (4) ein Druckregler (43) angeordnet ist, daß von der Gasableitung (5) eine Bypaßgasleitung (54) abzweigt, die mit der Wasserableitung (4) vor dem Druckregler (43) verbunden ist, daß die Probenleitung (41) von der Wasserableitung (4) vor dem Druckregler (43) abzweigt und daß der zweite Zweig der Gasableitung (5) für das nicht mehr benötigte Gas mit der Wasserableitung (4) hinter dem Druckregler (43) verbunden ist.

3. Einrichtung nach Anspruch 1,
**dadurch gekennzeichnet,**
daß die Wasserableitung (4) schleifenförmig nach der Art eines Siphons (44) angeordnet ist, wobei der mittlere Siphonschenkel (441) höher ist als eine Wassersäule, deren Druck der Differenz zwischen Außendruck und einem Unterdruck im Sammelbehälter (10) entspricht, und daß die Probenleitung (41) niveaugleich mit dem unteren Bogen (442) des Siphons (44) von der Wasserableitung (4) abzweigt.
4. Einrichtung nach Anspruch 1,
**dadurch gekennzeichnet,**
daß die Füllkörperkolonne (1), der Gastrockner, der Sammelbehälter (10) und die diese verbindenden Leitungen zum Abschirmen hinter einer vorhandenen Strahlenschutzwand (15) angeordnet sind und daß nur relativ dünne Zu-und Ableitungen für Gas und Wasser durch die Strahlenschutzwand (15) hindurchgeführt sind.
5. Einrichtung nach Anspruch 1,
**dadurch gekennzeichnet,**
daß der Gastrockner ein Gaskühler (6) ist, der zur Ableitung von Kondensat mit der Wasserableitung (4) in Verbindung steht.
6. Einrichtung nach den Ansprüchen 2 und 5,
**dadurch gekennzeichnet,**
daß der Gaskühler (6) zur Ableitung von Kondensat über die Bypaßgasleitung (54) mit der Wasserableitung (4) in Verbindung steht.
7. Einrichtung nach Anspruch 1,
**dadurch gekennzeichnet,**
daß in der rohrförmigen Füllkörperkolonne (1) im mittleren Bereich eines mit Füllkörpern gefüllten Rohrab schnittes (11) ein Trichter (12) angeordnet ist, dessen Öffnungsrand mit der Rohrinnenwand dicht abschließt.
8. Einrichtung nach Anspruch 1,
**dadurch gekennzeichnet,**
daß eines der Analysegeräte ein Gaschromatograph (7) mit Wärmeleitfähigkeitsdetektor ist.
9. Einrichtung nach Anspruch 1,
**dadurch gekennzeichnet,**
daß eines der Meßgeräte ein Strahlendetektor (8) zum Messen der Radioaktivität der Gase ist.
10. Einrichtung nach den Ansprüchen 8 und 9,
**dadurch gekennzeichnet,**
daß Gaschromatograph (7), Wärmeleitfähigkeitsdetektor und Strahlendetektor (8) hintereinander in Reihe angeordnet sind.

FIG 1

0 236 791

FIG 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE-A-2 363 370 (INTERATON INTERNATIONALE ATOMREATORBAU) * Seite 3, Zeilen 7-11; Seite 4, Zeilen 8-35; Seite 5, Zeilen 25-33; Patentansprüche 1,3; Abbildung 1 * | 1,2 | G 21 C 17/02 G 01 N 1/22 G 01 N 33/18 |
| A | FR-A-2 235 724 (COMMISSARIAT A L'ENERGIE ATOMIQUE) * Seite 2, Zeilen 17-26; Seite 3, Zeilen 12-15; Seite 4, Zeile 27 - Seite 5, Zeile 28; Seite 5, Zeilen 35-37; Abbildung 1 * | 1,9 | |
| A | DE-A-2 632 811 (BAYER AG) * Seite 3, Zeilen 17-30; Seite 4, Zeile 19 - Seite 5, Zeile 13; Abbildung 1 * | 1 | |
| | | | RECHERCHIERTE SACHGEBIETE (Int. Cl.4) |
| A | GB-A-2 158 235 (WATER RESEARCH CENTRE) * Seite 1, Zeilen 67-112; Patentansprüche 4,5; Abbildung * | 1 | G 01 N 1/00 G 21 C 17/00 B 01 D 19/00 G 01 N 33/00 |
| A | FR-A-2 099 682 (SIEMENS AG) | | |

Der vorliegende Recherchenbericht wurde für alle Patentansprüche erstellt.

| Recherchenort | Abschlußdatum der Recherche | Prüfer |
|---|---|---|
| DEN HAAG | 27-05-1987 | FORMBY N.M. |